# EUROPEAN PATENT APPLICATION

(11) **EP 1 505 065 A1**
(43) Date of publication of application: **09.02.2005**
(21) Application number: 03717676.5
(22) Date of filing: 21.04.2003
(51) Int. Cl.: C07D 301/26, C07D 303/36

(54) **PROCESS FOR PRODUCING AMINOEPOXIDE**

(30) Priority: 26.04.2002 JP 2002127579
(71) Applicant: Ajinomoto Co., Inc., Tokyo 104-8315 (JP)
(72) Inventor: OTAKE, Yasuyuki, c/o AJINOMOTO CO., INC., Kawasaki-shi, Kanagawa 210-8681 (JP); IZAWA, Kunisuke, c/o AJINOMOTO CO., INC., Kawasaki-shi, Kanagawa 210-8681 (JP)
(74) Representative: Nicholls, Kathryn Margaret
(86) International application number: PCT/JP2003/005069
(87) International publication number: WO 2003/091233

(57) **Abstract**

The present invention relates to a method of producing N-carbamate protected-3-amino-1,2-epoxy-4-(hydroxy substituted phenyl)butane at a high optical purity in a high yield, by hydrogenating N-carbamate protected-3-amino-1-chloro-2-hydroxy-4-(benzyloxy substituted phenyl)butane in the presence of a metal catalyst to give N-carbamate protected-3-amino-1-chloro-2-hydroxy-4-(hydroxy substituted phenyl)butane and treating this compound with a base.

## Description

### Technical Field

The present invention relates to a production method of optically active aminoepoxide represented by the following formula (1), which is useful as an intermediate compound for pharmaceutical products such as an HIV protease inhibitor and the like.

### Background Art

Aminoepoxide compounds represented by the formula (1) 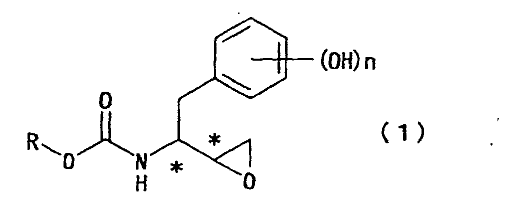 wherein R is an alkyl group or a fluorenylmethyl group, n is an integer of 1 or 2 and * is an asymmetric carbon atom independently showing an R or S configuration, are known to be useful as intermediate compounds for an HIV protease inhibitor (e.g., P. Chen et al., Journal of Medicinal Chemistry, Vol. 39, No. 10, page 1991, 1996).

As a production method of an aminoepoxide compound represented by the formula (1), for example, production methods depicted in the following Scheme 1 are disclosed in Journal of Organic Chemistry, Vol. 61, No. 11, page 3635, 1996) and WO99/10373. 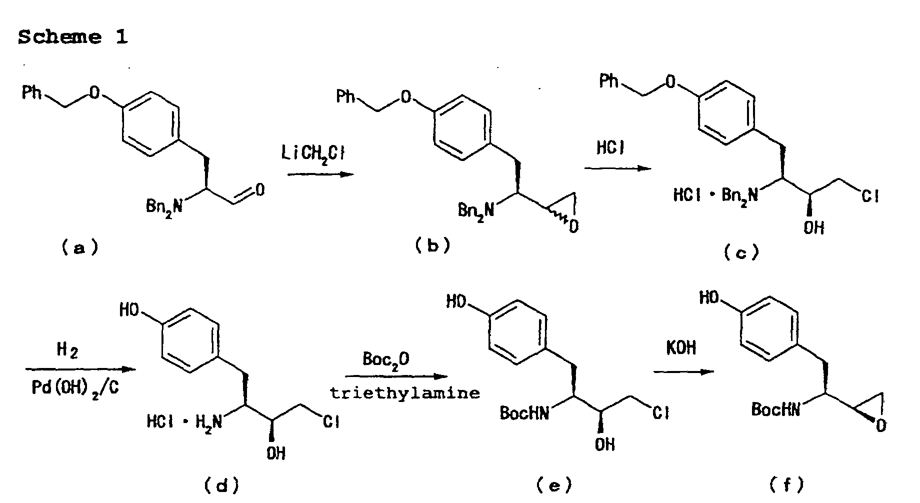 wherein Ph is a phenyl group, Bn is a benzyl group and Boc is a tert-butoxycarbonyl group.

The present method comprises removing a dibenzyl group, which is an amino-protecting group of compound (c), simultaneously with a benzyl group protecting a hydroxyl group, and protecting again the amino group with a carbamate protecting group. To stereoselectively obtain compound (c) having a (2S,3S) or (2R,3R) configuration as in the present method, the amino group is protected with a dibenzyl group and an aldehyde compound (a) is used as a starting material. However, since the aldehyde compound (a) is relatively easily racemized, close controls of the production conditions and step operations are required. Therefore, this method is not entirely a preferable method as a method for industrial production. As mentioned above, moreover, a step for converting an amino-protecting group is necessary. Furthermore, in the aforementioned reference J. Med. Chem., 1996, 39, 1991, the yield of aminoepoxide compound (f) is 93% by the reaction after deprotection of compound (c). In WO99/10373, however, the yield is not more than 50% by the same method.

Even when a debenzylation reaction of hydroxyl group is carried out under the conditions described in this reference and using a compound wherein amino group is protected by a tert-butoxycarbonyl group instead of the dibenzyl group of compound (c), deprotection (decarbamation) of amino group proceeds due to the acidic condition and a step for protecting the amino group again becomes necessary.

As a different production method of aminoepoxide compound (f), for example, Journal of Medicinal Chemistry, Vol. 39, No. 10, page 1991, 1996 discloses a production method shown in Scheme 2. 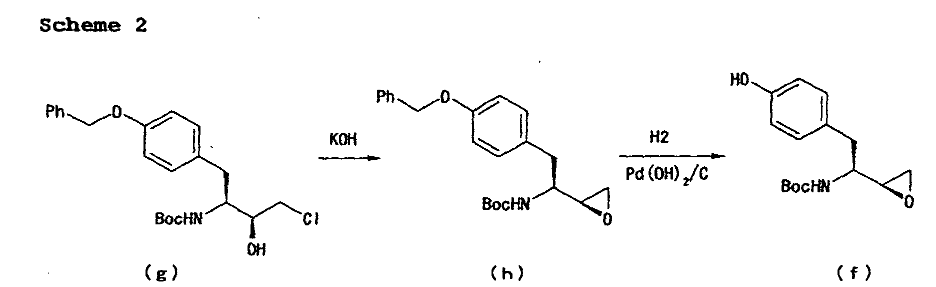 wherein Ph is a phenyl group and Boc is a tert-butoxycarbonyl group.

In the above-mentioned reference, the epoxide compound (h) was isolated and purified twice. In fact, when a debenzylation reaction was carried out without isolation and purification of compound (h), side reaction proceeded due to the impurity produced in the system and drastic degradation of quality and yield was confirmed. Since this kind of epoxide shows mutagenicity, isolation and purification of epoxide compound (h) require special facility. Therefore, this method is also hardly a method suitable for industrialization.

In addition, WO99/10373 discloses a production method shown in Scheme 3. 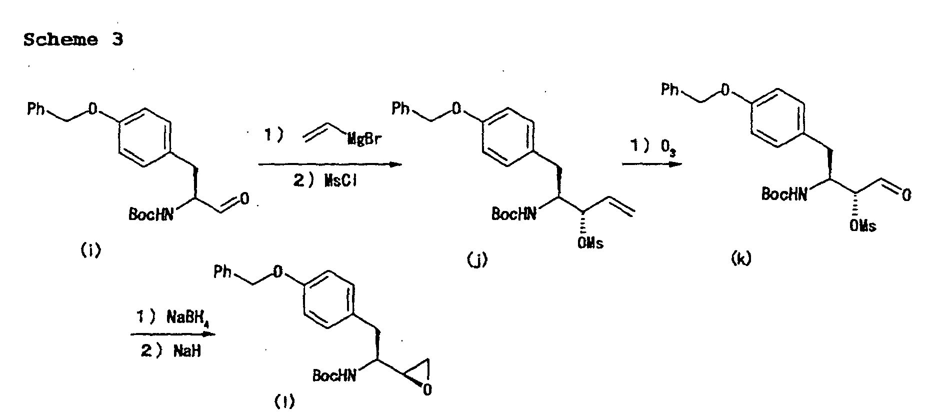 wherein Ph is a phenyl group, Boc is a tert-butoxycarbonyl group and Ms is a mesyl group.

Since the above-mentioned production method also comprises use of a more optically unstable aldehyde compound (i) than the aforementioned aldehyde compound (a) and includes a step of ozone oxidization, which is difficult to industrialize, this method is not entirely suitable for industrialization.

From the foregoing, the present invention aims at providing a method of producing aminoepoxide represented by the following formula (1) at a high optical purity in a high yield.

### Disclosure of the Invention

The present inventors have conducted intensive studies in an attempt to solve the aforementioned problems and found that hydrogenation of N-carbamate protected-3-amino-1-chloro-2-hydroxy-4-(benzyloxy-substituted phenyl)butane (the compound represented by the following formula (2)) in the presence of a metal catalyst affords N-carbamate protected-3-amino-1-chloro-2-hydroxy-4-(hydroxy-substituted phenyl)butane (the chlorohydrin compound represented by the following formula (3)), without confirmed elimination of chlorine by the reduction reaction. Furthermore, they have found that treatment of the obtained N-carbamate protected-3-amino-1-chloro-2-hydroxy-4-(hydroxy-substituted phenyl)butane with a base affords a highly optically pure N-carbamate protected-3-amino-1,2-epoxy-4-(hydroxy-substituted phenyl)butane (the aminoepoxide compound represented by the following formula (1)) in a high yield. The present inventors have completed the present invention based on these findings. Accordingly, the present invention encompasses the following.
[1] A production method of an aminoepoxide compound represented by the formula (1) 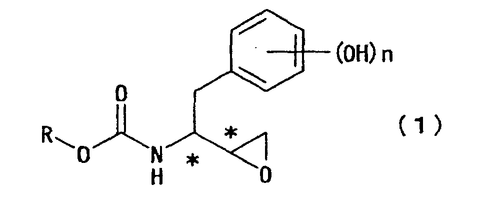 wherein R is an alkyl group or a fluorenylmethyl group, n is an integer of 1 or 2 and * is an asymmetric carbon atom independently showing an R or S configuration, which comprises hydrogenating a compound represented by the formula (2) 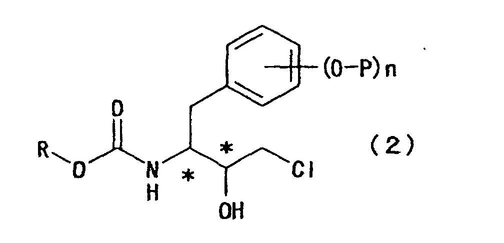 wherein R, n and * are as defined above and P is a benzyl group optionally having substituents, in the presence of a metal catalyst to give a chlorohydrin compound represented by the formula (3) 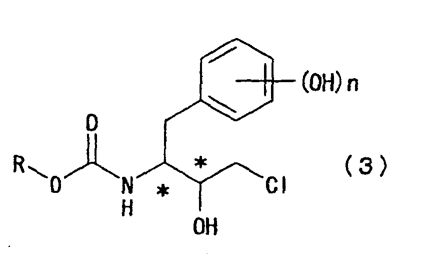 wherein R, n and * are as defined above, and treating the chlorohydrin compound with a base.
[2] The production method of [1], wherein configurations of the 2-position and the 3-position of the aminoepoxide compound represented by the formula (1), the compound represented by the formula (2) and the chlorohydrin compound represented by the formula (3) are each an S configuration or an R configuration.
[3] The production method of [1] or [2], wherein R is a tert-butoxycarbonyl group, n is 1 and the position of substitution on the aromatic ring is the 4-position.

When the configuration of the (2-position and 3-position) of an aminoepoxide compound represented by the formula (1) is (2S,3S), (2R,3R), (2R,3S) or (2S,3R), the configuration of the (2-position and 3-position) of a compound represented by the formula (2) is sequentially (2S,3S), (2R,3R), (2R,3S) or (2S,3R), and the configuration of the (2-position and 3-position) of a chlorohydrin compound represented by the formula (3) is sequentially (2S,3S), (2R,3R), (2R,3S) or (2S,3R).

The production method of the present invention is particularly preferably applied when the configurations of the 2-position and the 3-position of an aminoepoxide compound represented by the formula (1), a compound represented by the formula (2) and a chlorohydrin compound represented by the formula (3) are each an S configuration (i.e., (2S,3S)), or an R configuration (i.e., (2R,3R)).

### Mode of Embodiment of the Invention

The present invention is explained in detail in the following.

In the formula (1) and the formula (3), n is an integer of 1 or 2. That is, a compound represented by the formula (1) or the formula (2) is a compound having 1 or 2 hydroxyl groups on the phenyl group. A compound represented by the formula (1) or the formula (2) to be particularly preferably used in the present invention is particularly preferably used for a compound having a 4-hydroxyphenyl group (i.e., compound wherein n=1 and the position of substitution on aromatic ring is the 4-position).

In the formula (2), n is an integer of 1 or 2 and P is a benzyl group optionally having substituents. In other words, a compound represented by the formula (2) has 1 or 2 benzyloxy groups (aromatic ring of the benzyloxy group optionally has substituents) on a phenyl group.

The substituent when P has a substituent is not particularly limited as long as it is a group that does not exert an adverse influence on the reactions in the present invention and, for example, an alkoxy group (preferably having 1 to 7 carbon atoms), an alkyl group (preferably having 1 to 7 carbon atoms), a nitro group, a halogen group and the like can be mentioned. Generally, a compound wherein P is a benzyl group is preferably used. A compound represented by the formula (2), which is particularly preferably used in the present invention, is a compound having a 4-benzyloxyphenyl group (namely, n=1, P=benzyl group and the position of substitution on an aromatic ring is the 4-position).

In the formulas 1-3 of the present invention, R is an alkyl group or a fluorenylmethyl group. The alkyl group has 1 to 10 carbon atoms, and is preferably an alkyl group having 1 to 4 carbon atoms. For example, a methyl group, an ethyl group, a tert-butyl group and the like can be mentioned.

A compound represented by the formula (2), which is used as a starting material in the present invention, is a known compound, and can be produced by a known method such as reduction of a chloroketone compound represented by the formula (4) and the like (see WO00/44706, Europe Patent EP1081133). 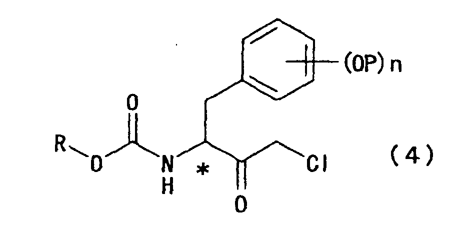 wherein R, P and n are as defined above, * is an asymmetric carbon atom, which means the compound independently has an R or S configuration.

In the following, a method of producing a chlorohydrin compound of the formula (3), which comprises hydrogenation (debenzylation) of a compound of the formula (2) in the presence of a metal catalyst, is explained.

As the metal catalyst, for example, palladium hydroxide on carbon, palladium carbon, Lindlar's catalyst and the like can be mentioned. Particularly, palladium hydroxide on carbon is preferable.

The amount of the metal catalyst to be used is not particularly limited and is preferably 0.0005-0.2 equivalent.

As the hydrogen source to be coexistent in the reaction system, for example, a hydrogen source to be generally used for debenzylation, such as hydrogen gas, cyclohexadiene, formic acid ammonium salt and the like, can be used. Preferred is hydrogen gas and cyclohexadiene, and particularly preferred is hydrogen gas.

The hydrogen pressure when hydrogen gas is used as a hydrogen source is preferably 1-10 atm, and particularly preferably 1-1.5 atm.

As the reaction solvent, for example, protic solvents such as methanol, ethanol, 1-propanol, 2-propanol, 1-butanol, water and the like, and aprotic solvents such as ethyl acetate, isopropyl acetate, acetone, 2-butanone, methylisobutylketone, tetrahydrofuran, 1,4-dioxane, toluene and the like can be preferably used. When the hydrogenate step and the following epoxidation reaction are to be conducted successively, the solvent to be used is 2-propanol or a mixed solvent of 2-propanol and water, which can be preferably used for the both steps. When the mixed solvent is used, the ratio of 2-propanol and water is preferably in the range of 2-propanol:water=1:1 - 100:1, more preferably in the range of 1:1 - 50:1. These reaction solvents may be used alone or in combination of one or more kinds thereof.

For hydrogenation, a base may be added to the reaction mixture to increase reaction rate. As the base to be used here includes, for example, lithium hydroxide, sodium hydroxide, potassium hydroxide, sodium carbonate, potassium carbonate, sodium hydrogen carbonate, potassium hydrogen carbonate and the like. Particularly preferred is sodium hydrogen carbonate. These bases may be added to the reaction mixture in the form of a solid, or may be used as an aqueous solution. While the amount of the base to be used varies depending on the base to be used, when the amount of the base is too large, the dechlorination reaction tends to proceed, and therefore, the amount is preferably not more than 1 equivalent, particularly preferably not more than 0.3 equivalent, relative to the amount of a compound represented by the formula (2) to be used.

While the reaction temperature also varies depending on the kind of the solvent and the amount of the base to be used, it is generally 20°C to 80°C, preferably 40°C to 60°C. The reaction temperature may be changed during the course of the reaction. While the reaction time is not particularly limited, it is preferably about 30 min - 24 hr.

The reaction is generally conducted under stirring. After the completion of the reaction, the metal catalyst is removed from the reaction mixture by filtration and the like to give a solution containing a chlorohydrin compound represented by the formula (3). In this case, the chlorohydrin compound represented by the formula (3) can be continuously subjected to an epoxidation reaction in the next step, without isolation.

The solubility of a chlorohydrin compound represented by the formula (3) in a reaction mixture can be increased by basifying the reaction solution by adding a base before filtering off the metal catalyst. When a metal catalyst is filtered off, therefore, chlorohydrin compound can be prevented from being precipitated and simultaneously filtered off. In this case, the chlorohydrin compound is partially or entirely converted to aminoepoxide represented by the formula (1). As the base to be used, aqueous lithium hydroxide solution, aqueous sodium hydroxide solution, aqueous potassium hydroxide solution, aqueous sodium carbonate solution, aqueous potassium carbonate solution, aqueous sodium hydrogen carbonate solution, aqueous potassium hydrogen carbonate solution and the like can be mentioned. Particularly preferred is aqueous sodium hydroxide solution. The amount of the base to be used is preferably 0.1-5.0 equivalents, particularly preferably 0.1-3.0 equivalents, relative to the amount of the compound represented by the formula (2) used in the hydrogenate step. When a base has been already used in the hydrogenate step, the difference between the above-mentioned amount of the base and the amount of the base already used can be added.

To increase the yield, a metal catalyst filtered off may be washed with a mixed solvent of an organic solvent and a base and the washing may be added to the above-mentioned solution.

As the organic solvent to be used for washing the metal catalyst, protic solvents such as methanol, ethanol, 1-propanol, 2-propanol, 1-butanol, water and the like, or aprotic solvents such as ethyl acetate, isopropyl acetate, acetone, 2-butanone, methyl isobutyl ketone, tetrahydrofuran, 1,4-dioxane, toluene and the like can be preferably used. Particularly preferred is 2-propanol.

As a base to be mixed with an organic solvent for washing the metal catalyst, aqueous lithium hydroxide solution, aqueous sodium hydroxide solution, aqueous potassium hydroxide solution, aqueous sodium carbonate solution, aqueous potassium carbonate solution, aqueous sodium hydrogen carbonate solution, aqueous potassium hydrogen carbonate solution and the like can be mentioned. Particularly preferred is aqueous sodium hydroxide solution. The amount of the base to be used including the amount of a base already used is preferably within the range not exceeding 5.0 equivalents, particularly preferably 3.0 equivalents, relative to the amount of a compound represented by the formula (2).

A chlorohydrin compound represented by the formula (3) can be continuously subjected to the epoxidation reaction in the next step without isolation. As a reaction solvent in this case, 2-propanol or a mixed solvent of 2-propanol and water, which can be also preferably used in the hydrogenate step, can be used.

A method of obtaining an aminoepoxide compound represented by the formula (1) by reacting a base with a chlorohydrin compound represented by the formula (3) is explained in the following.

While an organic solvent to be used for the reaction is not particularly limited as long as it does not exert an adverse influence on the reaction, for example, methanol, ethanol, 1-propanol, 2-propanol, 1-butanol, water and the like can be preferably used. Particularly, 2-propanol and a mixed solvent of 2-propanol and water are used. These reaction solvents may be used alone or in combination of one or more kinds thereof.

As the base to be used, aqueous lithium hydroxide solution, aqueous sodium hydroxide solution, aqueous potassium hydroxide solution, aqueous sodium carbonate solution, aqueous potassium carbonate solution, aqueous sodium hydrogen carbonate solution, aqueous potassium hydrogen carbonate solution and the like can be mentioned. Particularly preferred is aqueous sodium hydroxide solution.

The amount of the base to be used is generally 2.0-5.0 equivalents, preferably 2.0-3.0 equivalents. When a base has been already added in a hydrogenation step, a step of filtering a metal catalyst and the like, a base can be used in the above-mentioned amount less the amount of the base already used.

After the completion of the reaction, an excess base is neutralized with an acid. As the acid to be used here, for example, hydrochloric acid, sulfuric acid, nitric acid, acetic acid, citric acid and the like can be mentioned. Particularly preferred is citric acid.

While the amount of the acid to be used varies depending on the amount of the base to be used, for example, it is preferably 1.0-3.0 equivalents when the amount of the base to be used is 2.0-3.0 equivalents.

The reaction is generally carried out with stirring, and the reaction temperature is generally preferably -10°C to 40°C, particularly preferably 0°C to 25°C. The reaction temperature may be changed during the course of the reaction. While the reaction time is not particularly limited, it is preferably about 30 min - 24 hr.

The reaction mixture is crystallized by concentrating as necessary, or the solvent is completely evaporated from the reaction mixture as necessary and a different solvent is used to allow crystallization to give aminoepoxide represented by the formula (1) as crystals.

When the configuration is an S configuration (i.e., (2S,3S)) or an R configuration (i.e., (2R,3R) for both the 2-position and the 3-position, the solvent to be used for the crystallization is preferably a mixed solvent of a protic solvent such as methanol, ethanol, 1-propanol, 2-propanol, 1-butanol and the like and water, and a mixed solvent of 2-propanol and water is particularly preferable. As the temperature of crystallization, -10°C to 20°C is preferable. For example, when these solvents have been used as a reaction solvent for the epoxidation reaction, crystallization can be performed directly (or after concentration as necessary).

The aminoepoxide compound represented by the formula (1) obtained in this way can be also subjected to protection of hydroxyl group on an aromatic ring with a suitable protecting group as necessary.

Since the series of reactions explained above proceed stereoselectively, the object compound can be obtained at a high optical purity in a high yield. As one example of preferable embodiments of the production method of the present invention, a series of reaction schemes for producing (2S,3S)-N-carbamate protected-3-amino-1,2-epoxy-4-(4-hydroxyphenyl)butane using (2S,3S)-N-carbamate protected-3-amino-1-chloro-2-hydroxy-4-(4-benzyloxyphenyl)butane are shown in the following. 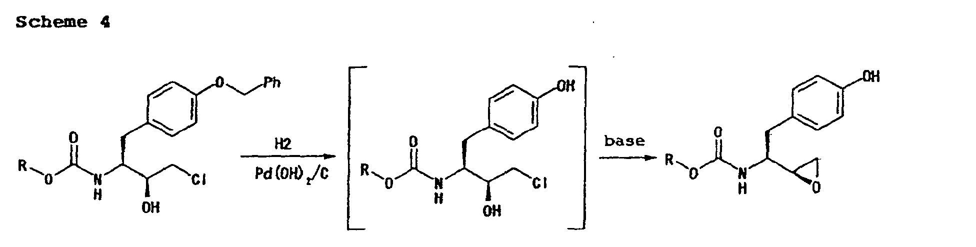 wherein R is as defined above. One example of preferable embodiments is a tert-butyl group.

### Examples

The present invention is explained in more detail in the following by referring to Examples, which are not to be construed as limitative in any way.

### <Reference Example 1>

### Production of methyl 2R-amino-3-(4-benzyloxyphenyl)propionate hydrochloride

1N Aqueous sodium hydroxide solution (26.2 ml) and copper sulfate (2.09 g) were added to 2R-amino-3-(4-hydroxyphenyl)propionic acid (4.75 g) and the mixture was stirred at room temperature for 1 hr. To this solution were added methanol (158 ml) and 2N aqueous sodium hydroxide solution (13.1 ml), and then benzyl bromide (3.3 ml) was added slowly. The mixture was stirred at room temperature for 3 hr. The product was collected by filtration and the crystals were washed with a mixed solvent of methanol (12.5 ml) and water (12.5 ml). 1N Aqueous hydrochloric acid solution (30 ml) was added to the obtained crystals and the mixture was stirred at 50°C for 30 min. The crystals were collected by filtration and 1N aqueous hydrochloric acid solution (30 ml) was added to the obtained crystals. The mixture was stirred at 50°C for 30 min and the crystals were collected by filtration. The obtained crystals were washed with water (25 ml), after which water (30 ml) was added. The mixture was adjusted to pH 6.3 with sodium hydroxide and the crystals were collected by filtration. The obtained crystals were dried to give 2R-amino-3-(4-benzyloxyphenyl)propionic acid as crystals (3.24 g, yield 46%). Thionyl chloride (0.959 ml) was added to methanol (32.4 ml) at 0°C, and the obtained crystals (3.24 g) of 2R-amino-3-(4-benzyloxyphenyl)propionic acid were added to this solution. The mixture was stirred at 60°C for 15 hr. This reaction mixture was concentrated under reduced pressure and ethyl acetate (65 ml) was added to allow crystallization. The obtained crystals were collected by filtration and dried under reduced pressure to give the object methyl 2R-amino-3-(4-benzyloxyphenyl)propionate hydrochloride (3.30 g, yield by crystallization 97%, yield relative to 2R-amino-3-(4-hydroxyphenyl)propionic acid 44%).
¹H-NMR(CD₃OD, 400 MHz) δppm: 3.12 (dd, 1H, J = 14.4, 7.3 Hz), 3.19 (dd, 1H,J = 14.4, 6.0 Hz), 3.79 (s, 3H), 4.27 (dd, 1H, J = 7.4, 6.0 Hz), 4.85 (s, 3H), 5.08 (s, 2H), 6.98-7.01 (m, 2H), 7.16-7.23 (m, 2H), 7.26-7.45 (m, 5H) ¹³C-NMR(CD₃OD, 400 MHz) δppm: 37.0, 54.0, 55.7, 71.4, 117.0, 127.7, 129.0, 129.3, 129.9, 132.0, 139.0, 160.3, 170.9
mass spectrum m/e: 286 (M-Cl)⁺

### <Reference Example 2>

### Production of methyl 2S-N-phenylmethylene-2-amino-3-(4-benzyloxyphenyl)propionate

To methyl 2S-amino-3-(4-benzyloxyphenyl)propionate hydrochloride (5.15 g, manufactured by KOKUSAN CHEMICAL Co., Ltd.) was added dichloromethane (25.8 ml) under an argon atmosphere, and the mixture was stirred at 0°C. Sodium sulfate (4.55 g), benzaldehyde (1.63 ml) and triethylamine (2.23 ml) were added and the mixture was stirred at 0°C for 23 hr. Sodium sulfate was filtered off and the obtained solution was concentrated under reduced pressure. t-Butyl methyl ether (50 ml) was added and insoluble materials were filtered off. The obtained solution was concentrated under reduced pressure to give the object methyl 2S-N-phenylmethylene-2-amino-3-(4-benzyloxyphenyl)propionate (5.16 g, yield 86%).
¹H-NMR(CDCl₃, 400 MHz) δppm: 3.08 (dd, 1H, J = 13.6, 8.0 Hz), 3.30 (dd, 1H,J = 13.6, 5.2 Hz), 3.74 (s, 3H), 4.13 (dd, 1H, J = 8.8, 5.2 Hz), 4.99 (s, 2H), 6.83-7.72 (m, 14H), 7.92 (s, 1H)
¹³C-NMR(CDCl₃, 400 MHz)δppm: 39.4, 52.6, 70.4, 75.7, 115.1, 115.4, 127.9, 128.3, 128.9, 128.9, 130.1, 131.2, 131.5, 136.0, 137.5, 157.9, 164.1, 172.6
mass spectrum m/e: 374 (M+H)⁺

### <Reference Example 3>

### Production of methyl 2R-N-phenylmethylene-2-amino-3-(4-benzyloxyphenyl)propionate

To methyl 2R-amino-3-(4-benzyloxyphenyl)propionate hydrochloride (3.20 g) obtained in Reference Example 1 was added dichloromethane (8.64 ml) under an argon atmosphere and the mixture was stirred at 5°C. Sodium sulfate (2.83 g), benzaldehyde (1.01 ml) and triethylamine (1.39 ml) were added and the mixture was stirred at 5°C for 18 hr. Sodium sulfate was filtered off and the obtained solution was concentrated under reduced pressure. t-Butyl methyl ether (31 ml) was added and insoluble materials were filtered off. t-Butyl methyl ether (31 ml) was added and insoluble materials were filtered off. The obtained solution was concentrated under reduced pressure to give the object methyl 2S-N-phenylmethylene-2-amino-3-(4-benzyloxyphenyl)propionate (3.01 g, yield 82%).

### <Reference Example 4>

### Production of 3S-amino-1-chloro-4-(4-benzyloxyphenyl)-2-butanone hydrochloride

To methyl 2S-N-phenylmethylene-2-amino-3-(4-benzyloxyphenyl)propionate (4.0 g) obtained in the same manner as in Reference Example 2 were added bromochloromethane (0.905 ml), tetrahydrofuran (15 ml) and toluene (15 ml) and the mixture was stirred under an argon atmosphere at -78°C. A solution (2.66 M, 5.23 ml) of normal butyl lithium in hexane was slowly added dropwise over 1.5 hr. After the completion of the dropwise addition, the mixture was further stirred for 30 min and the reaction mixture was poured at once into a mixed solution of 36% aqueous hydrochloric acid solution (2.39 ml) and methanol (5.40 ml) to quench the reaction. This solution was stirred at 25°C for 1 hr and the resulting crystals were collected by filtration. The obtained crystals were washed with hexane (10 ml) and dried under reduced pressure to give 3S-amino-1-chloro-4-(4-benzyloxyphenyl)-2-butanone hydrochloride (2.65 g, yield 73%).
¹H-NMR(DMSO-d₆,400 MHz) δppm: 2.84-3.14 (m, 2H), 4.43-4.50 (m, 1H), 4.52 (d, 1H, J = 17.4 Hz), 4.74 (d, 1H, J = 17.4 Hz), 5.08 (s, 2H), 6.94-7.00 (m, 2H), 7.21-7.25 (m, 2H), 7.30-7.53 (m, 5H), 8.73 (bs, 3H)
¹³C-NMR(DMSO-d₆, 400 MHz) δppm: 34.5, 48.3, 57.7, 69.5, 115.0, 126.8, 128.0, 128.2, 128.8, 131.0, 137.4, 158.0, 198.6
mass spectrum m/e: 304 (M-Cl)⁺

### <Reference Example 5>

### Production of 3R-amino-1-chloro-4-(4-benzyloxyphenyl)-2-butanone hydrochloride

To methyl 2R-N-phenylmethylene-2-amino-3-(4-benzyloxyphenyl)propionate (3.0 g) obtained in the same manner as in Reference Example 3 were added bromochloromethane (0.680 ml), tetrahydrofuran (11.3 ml) and toluene (11.3 ml), and the mixture was stirred under an argon atmosphere at -78°C. A solution (2.66 M, 3.92 ml) of normal butyl lithium in hexane was slowly added dropwise over 2 hr. After the completion of the dropwise addition, the mixture was further stirred for 30 min and the reaction mixture was poured at once into a mixed solution of 36% aqueous hydrochloric acid solution (1.79 ml) and methanol (4.05 ml) to quench the reaction. This solution was stirred at 25°C for 1 hr and at 0°C for 1 hr. The resulting crystals were collected by filtration and washed with hexane (7.5 ml). The crystals were dried under reduced pressure to give 3R-amino-1-chloro-4-(4-benzyloxyphenyl)-2-butanone hydrochloride (2.65 g, yield 97%).

### <Reference Example 6>

### Production of (2S,3S)-N-tert-butoxycarbonyl-3-amino-4-(4-benzyloxyphenyl)-1-chloro-2-hydroxybutane

di-tert-Butoxycarbonate (10.4 g), potassium carbonate (6.58 g), dichloromethane (135 ml) and water (67.5 ml) were stirred at 35°C and 3S-amino-1-chloro-4-(4-benzyloxyphenyl)-2-butanone hydrochloride (13.5 g) obtained in the same manner as in Reference Example 4 was added. This reaction mixture was stirred at 35°C for 2 hr. The organic layer and the aqueous layer were separated and the organic layer was washed with 1N aqueous hydrochloric acid solution (54 ml) and water (54 ml). The obtained organic layer was concentrated under reduced pressure and ethanol (30 ml) was added. The mixture was concentrated again under reduced pressure. Ethanol (144 ml) was added and sodium borohydride (0.647 g) was added in several portions at room temperature. After stirring at room temperature for 2 hr, sodium borohydride (0.065 g) was further added and the mixture was stirred at room temperature for 1 hr. The reaction was quenched using acetic acid (1.12 ml) and the reaction mixture was heated to 70°C. This solution was allowed to cool to 20°C over 5 hr and the mixture was stirred at 20°C for 10 hr. The obtained crystals were collected by filtration and the crystals were washed with ethanol (22 ml). The crystals were further washed twice with water (45 ml, 45 ml). The obtained crystals were dried under reduced pressure to give the object (2S,3S)-N-tert-butoxycarbonyl-3-amino-4-(4-benzyloxyphenyl)-1-chloro-2-hydroxybutane (9.50 g, yield 59%). The diastereoselectivity then was (2S,3S)/(2R,3S)=98.9/1.1.
¹H-NMR (DMSO-d₆, 400 MHz) δppm: 1.28 (s, 9H), 2.88-2.47 (m, 1H), 3.35-3.70 (m, 5H), 5.05 (s, 2H), 5.41 (d, 1H, J = 6.0 Hz), 6.65 (d, 1H, J = 8.4 Hz), 6.89 (d, 2H, J = 8.8 Hz), 7.08 (d, 2H, J = 8.8 Hz), 7.30-7.45 (m, 5H)
¹³C-NMR(DMSO-d₆, 400 MHz) δppm: 28.5, 35.0, 48.4, 54.9, 69.5, 73.1, 77.9, 114.7, 127.9, 128.1, 128.7, 130.5, 131.6, 137.7, 155.5, 157.0
mass spectrum m/e: 406 (M+H)⁺
[α]²⁰_{D} = 7.0° (c=1.0, CH₂Cl₂)
melting point: 161-162°C

### <Reference Example 7>

### Production of (2R,3R)-N-tert-butoxycarbonyl-3-amino-4-(4-benzyloxyphenyl)-1-chloro-2-hydroxybutane

di-tert-Butoxycarbonate (1.92 g), potassium carbonate (1.22 g), dichloromethane (25 ml) and water (12.5 ml) were stirred at 35°C and 3R-amino-1-chloro-4-(4-benzyloxyphenyl)-2-butanone hydrochloride (2.50 g) obtained in the same manner as in Reference Example 5 was added. This reaction mixture was stirred at 35°C for 1.5 hr. The organic layer and the aqueous layer were separated and the organic layer was washed with 1N aqueous hydrochloric acid solution (10 ml) and water (10 ml). The obtained organic layer was concentrated under reduced pressure and ethanol (5.5 ml) was added. The mixture was concentrated again under reduced pressure. Ethanol (27.2 ml) was added and sodium borohydride (0.128 g) was added in several portions at room temperature. After stirring at 25°C for 1 hr, the reaction was quenched using acetic acid (0.193 ml) and the reaction mixture was heated to 70°C. This solution was allowed to cool to 20°C over 5 hr and the mixture was stirred at 20°C for 12 hr. The obtained crystals were collected by filtration and crystals were washed with ethanol (4.1 ml). The crystals were further washed twice with water (8.2 ml, 8.2 ml). The obtained crystals were dried under reduced pressure to give the object (2R,3R)-N-tert-butoxycarbonyl-3-amino-4-(4-benzyloxyphenyl)-1-chloro-2-hydroxybutane (1.80 g, yield 60%). The diastereoselectivity then was (2R,3R)/(2S,3R)=99.0/1.0.

### <Example 1>

### Production of (2S,3S)-N-tert-butoxycarbonyl-3-amino-4-(4-hydroxyphenyl)-1-chloro-2-hydroxybutane

Ethanol (2.5 ml) was added to (2S,3S)-N-tert-butoxycarbonyl-3-amino-4-(4-benzyloxyphenyl)-1-chloro-2-hydroxybutane (0.250 g) obtained in the same manner as in Reference Example 6. 20% palladium hydroxide on carbon (0.0025 g, manufactured by Kawaken Fine Chemicals Co., Ltd., the moisture content 49.1%) was further added under an argon atmosphere and the mixture was stirred under a hydrogen atmosphere (1 atm) at 25°C for 3 hr. After stirring further at 40°C for 3 hr, palladium hydroxide on carbon was filtered off. The obtained solution was concentrated under reduced pressure to give the object (2S,3S)-N-tert-butoxycarbonyl-3-amino-4-(4-hydroxyphenyl)-1-chloro-2-hydroxybutane (0.191 g, yield 98%).
¹H-NMR(DMSO-d₆, 400 MHz) δppm: 1.29 (s, 9H) , 2.46 (dd, 1H, J = 14.0, 9.6 Hz), 2.83-2.92 (m, 1H), 3.30-3.65 (m, 5H), 6.62-6.68 (m, 3H), 6.94 (d, 2H, J = 8.4 Hz)
¹³C-NMR (DMSO-d₆, 400 MHz) δppm: 28.6, 34.9, 48.4, 54.9, 73.1, 77.8, 115.1, 129.3, 130.3, 155.5, 155.9
mass spectrum m/e: 314 (M-H)⁻

### <Example 2>

### Production of (2S,3S)-N-tert-butoxycarbonyl-3-amino-1,2-epoxy-4-(4-hydroxyphenyl)butane

2-Propanol (38 ml) was added to (2S,3S)-N-tert-butoxycarbonyl-3-amino-4-(4-benzyloxyphenyl)-1-chloro-2-hydroxybutane (3.83 g) obtained in the same manner as in Reference Example 6. 20% palladium hydroxide on carbon (0.192 g, manufactured by Kawaken Fine Chemicals Co., Ltd., moisture content 49.1%) was further added under an argon atmosphere, and the mixture was stirred under a hydrogen atmosphere (1 atm) at 60°C for 1.5 hr. This reaction mixture was allowed to cool to 20°C and 6N aqueous sodium hydroxide solution (2.93 ml) was added. Palladium hydroxide on carbon was filtered off and the filtered palladium hydroxide on carbon was washed with a mixed solution of 2-propanol (7.6 ml) and 6N aqueous sodium hydroxide solution (1.47 ml). The palladium hydroxide on carbon was filtered off and the obtained mother liquor and the washing were stirred at 20°C for 2 hr. 6N Aqueous sodium hydroxide solution (0.146 ml) was added and the mixture was stirred for 1 hr. Water (41.4 ml) and citric acid (1.77 g) were added to the reaction mixture to quench the reaction. This solution was cooled to 0°C over 3 hr and further stirred at 0°C for 7 hr. The obtained crystals were collected by filtration and washed with a mixed solution of 2-propanol (3.8 ml) and water (11.5 ml). The crystals were washed twice with water (38 ml, 38 ml). The obtained crystals were dried under reduced pressure to give the object (2S,3S)-N-tert-butoxycarbonyl-3-amino-1,2-epoxy-4-(4-hydroxyphenyl)butane (2.39 g, yield 91%). Chiral analysis by HPLC confirmed an optical purity to be not less than 99.5%.
¹H-NMR(DMSO-d₆, 400 MHz) δppm: 1.30 (s, 9H), 2.54-2.68 (m, 3H), 2.73 (dd, 1H, J = 14.0, 4.4 Hz), 2.85-2.89 (m, 1H), 3.37-3.45 (m, 1H), 6.64 (d, 2H, J = 8.4 Hz), 6.78 (d, 1H, J = 8.8 Hz), 6.98 (d, 2H, J = 8.4 Hz), 9.14 (s, 1H)
¹³C-NMR(DMSO-d₆, 400 MHz) δppm: 28.5, 36.6, 44.9, 53.4, 53.6, 78.0, 115.2, 128.7, 130.3, 155.6, 155.9 mass spectrum m/e: 280 (M+H)⁺
[α]²⁰_{D} =-3.8° (c=1.0, MeOH)
melting point: 160-161°C

### <Example 3>

### Production of (2R,3R)-N-tert-butoxycarbonyl-3-amino-1,2-epoxy-4-(4-hydroxyphenyl)butane

2-Propanol (17 ml) was added to (2R,3R)-N-tert-butoxycarbonyl-3-amino-4-(4-benzyloxyphenyl)-1-chloro-2-hydroxybutane (1.70 g) obtained in the same manner as in Reference Example 7. 20% palladium hydroxide on carbon (0.085 g, manufactured by Kawaken Fine Chemicals Co., Ltd., moisture content 49.1%) was further added under an argon atmosphere, and the mixture was stirred under a hydrogen atmosphere (1 atm) at 60°C for 2 hr. This reaction mixture was allowed to cool to 20°C and 6N aqueous sodium hydroxide solution (1.40 ml) was added. Palladium hydroxide on carbon was filtered off and the filtered palladium hydroxide on carbon was washed with a mixed solution of 2-propanol (3.4 ml) and 6N aqueous sodium hydroxide solution (0.698 ml). The palladium hydroxide on carbon was filtered off and the obtained mother liquor and the washing were stirred at 20°C for 1.5 hr. Water (18.3 ml) and citric acid (0.805 g) were added to the reaction mixture to quench the reaction. This solution was cooled to 0°C over 3 hr and further stirred at 0°C for 12 hr. The obtained crystals were collected by filtration and washed with a mixed solution of 2-propanol (1.7 ml) and water (5.1 ml). The crystals were washed twice with water (17 ml, 17 ml). The obtained crystals were dried under reduced pressure to give the object (2R,3R)-N-tert-butoxycarbonyl-3-amino-1,2-epoxy-4-(4-hydroxyphenyl)butane (1.07 g, yield 91%).

### <Reference Example 8>

### Production of (2S,3S)-N-tert-butoxycarbonyl-3-amino-4-(4-benzyloxyphenyl)-1-chloro-2-hydroxybutane

The mother liquor resulting from collection of crystals by filtration in Reference Example 5 was analyzed to find that 2.97 g of N-tert-butoxycarbonyl-3-amino-4-(4-benzyloxyphenyl)-1-chloro-2-hydroxybutane ((2S,3S)/(2R,3S)=42.0/58.0) was contained. This solution was concentrated under reduced pressure. Water (50 ml) was added to the obtained crystals and the mixture was stirred at room temperature for 1 hr. The crystals were collected by filtration. A mixed solution of hexane (7.5 ml) and ethyl acetate (7.5 ml) was added and the mixture was stirred at room temperature for 1 hr. The crystals were collected by filtration and dried under reduced pressure to give (2S,3S)-N-tert-butoxycarbonyl-3-amino-4-(4-benzyloxyphenyl)-1-chloro-2-hydroxybutane (1.05 g, recovery yield 35.3%, (2S,3S)/(2R,3S)=84.5/15.5).

### <Example 4>

### Production of (2S,3S)-N-tert-butoxycarbonyl-3-amino-1,2-epoxy-4-(4-hydroxyphenyl)butane

2-Propanol (9.2 ml) and 5% aqueous sodium hydrogen carbonate solution (0.379 mg) were added to (2S,3S)-N-tert-butoxycarbonyl-3-amino-4-(4-benzyloxyphenyl)-1-chloro-2-hydroxybutane (0.915 g, (2S,3S)/(2R,3S)=84.5/15.5) obtained in the same manner as in Reference Example 8. 20% palladium hydroxide on carbon (0.046 g, manufactured by Kawaken Fine Chemicals Co., Ltd., moisture content 49.1%) was added under an argon atmosphere and the mixture was stirred under a hydrogen atmosphere at 40°C for 30 min. Palladium hydroxide on carbon was filtered off and the filtered palladium hydroxide on carbon was washed with a mixed solution of 2-propanol (1.8 ml) and 6N aqueous sodium hydroxide solution (1.08 ml). The obtained reaction mixture was stirred at 20°C for 2 hr. Water (9.4 ml) and citric acid (0.433 g) were added to this reaction mixture to quench the reaction. This reaction mixture was stirred at 0°C for 1 hr and the obtained crystals were collected by filtration. The crystals were washed with a mixed solution of 2-propanol (0.75 ml) and water (2.3 ml) and then further washed with water (9.2 ml, 9.2 ml). The obtained crystals were dried under reduced pressure to give the object (2S,3S)-N-tert-butoxycarbonyl-3-amino-1,2-epoxy-4-(4-hydroxyphenyl)butane (0.466 g, yield 74%, (2S,3S)/(2R,3S)=97.7/2.3).

### <Comparative Example 1>

2-Propanol (1.86 ml) and 6N aqueous sodium hydroxide solution (0.058 ml) were added to (2R,3R)-N-tert-butoxycarbonyl-3-amino-4-(4-benzyloxyphenyl)-1-chloro-2-hydroxybutane (93.4 g) obtained in the same manner as in Reference Example 7 and the mixture was stirred at 20°C for 1 hr. Consumption of the starting material was confirmed and citric acid (7.4 mg) was added to quench the reaction. 20% palladium hydroxide on carbon (4.7 mg, manufactured by Kawaken Fine Chemicals Co., Ltd., moisture content 49.1%) and water (0.560 ml) were added under an argon atmosphere, and the mixture was stirred under a hydrogen atmosphere at 60°C. After 1 hr, the reaction was analyzed by HPLC to confirm that debenzylation of an intermediate (2R,3R)-N-tert-butoxycarbonyl-3-amino-1,2-epoxy-4-(4-benzyloxyphenyl)butane proceeded to give the object (2R,3R)-N-tert-butoxycarbonyl-3-amino-1,2-epoxy-4-(4-hydroxyphenyl)butane (76.2% by area). After further stirring for 16 hr, the reaction was analyzed by HPLC to find that the object (2R,3R)-N-tert-butoxycarbonyl-3-amino-1,2-epoxy-4-(4-hydroxyphenyl)butane was 9.5% by area, thus confirming a number of impurities. The results reveal that debenzylation of epoxy compound after synthesis makes control of debenzylation reaction difficult.

### Industrial Applicability

According to the present invention, aminoepoxide represented by the above-mentioned formula (1) can be produced at a high optical purity in a high yield.

This application is based on a patent application No. 127579/2002 filed in Japan, the contents of which are hereby incorporated by reference.

## Claims

1. A production method of an aminoepoxide compound represented by the formula (1) 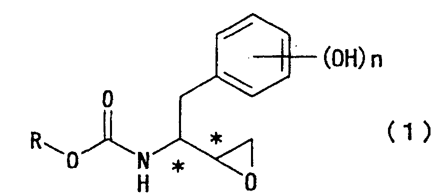 wherein R is an alkyl group or a fluorenylmethyl group, n is an integer of 1 or 2 and * is an asymmetric carbon atom independently showing an R or S configuration, which comprises hydrogenating a compound represented by the formula (2) 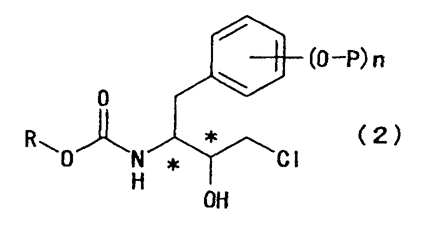 wherein R, n and * are as defined above and P is a benzyl group optionally having substituents, in the presence of a metal catalyst to give a chlorohydrin compound represented by the formula (3) 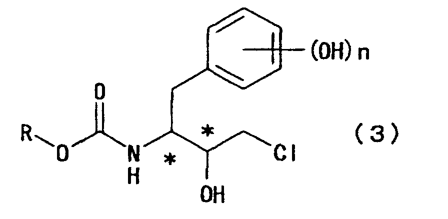 wherein R, n and * are as defined above, and treating the chlorohydrin compound with a base.

2. The production method of claim 1, wherein configurations of the 2-position and the 3-position of the aminoepoxide compound represented by the formula (1), the compound represented by the formula (2) and the chlorohydrin compound represented by the formula (3) are each an S configuration or an R configuration.

3. The production method of claim 1 or 2, wherein R is a tert-butoxycarbonyl group, n is 1 and the position of substitution on the aromatic ring is the 4-position.
